# EUROPÄISCHE PATENTSCHRIFT

(11) **EP 0 866 804 B1**
(45) Veröffentlichungstag und Bekanntmachung des Hinweises auf die Patenterteilung: **19.09.2001**
(21) Anmeldenummer: 96942336.7
(22) Anmeldetag: 04.12.1996
(51) Int. Cl.: C07K 14/46, A61K 38/17, A61K 7/48, A61K 7/06

(54) **GEGEN BAKTERIEN, MYCOTA UND VIREN WIRKSAME VERTEBRATEN PROTEINE UND DESSEN KOSMETISCHE ZUBEREITUNGEN**
COSMETIC PREPARATIONS CONTAINING VERTEBRATE PROTEINS AND HAVING ANTIBACTERIAL, ANTIMYCOTICAL AND ANTIVIRAL ACTION
PREPARATIONS COSMETIQUES RENFERMANT DES PROTEINES DE VERTEBRES, A EFFET ANTIBACTERIEN, ANTIMYCOSIQUE ET ANTIVIRAL

(30) Priorität: 16.12.1995 DE 19547162; 19.07.1996 DE 19629119
(43) Veröffentlichungstag der Anmeldung: 30.09.1998
(73) Patentinhaber: Planton GmbH,, 24105 Kiel (DE)
(72) Erfinder: CHRISTOPHERS, ENNO, D-24105 Kiel (DE); HOPPE, Udo, D-22397 Hamburg (DE); SCHRÖDER, Jens-M., D-24241 Blumenthal (DE); WOLF, Florian, D-20251 Hamburg (DE)
(74) Vertreter: Dehmel, Albrecht, Dr.
(86) Internationale Anmeldenummer: EP9605405
(87) Internationale Veröffentlichungsnummer: WO9722624

(56) Entgegenhaltungen:
- WO-A-94/21672

## Beschreibung

Die vorliegende Erfindung betrifft die Verwendung bestimmter Vertebratenproteine als gegen Bakterien, Mycota und Viren wirksame Substanzen. In besonderen Ausführungsformen betrifft die vorliegende Erfindung kosmetische und dermatologische Zubereitungen, solche Substanzen enthaltend.

Der gesunde warmblütige Organismus, insbesondere die gesunde menschliche Haut, ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bakterien gehören zu den prokaryotischen Einzellern. Sie können grob nach ihrer Form (Kugel, Zylinder, gekrümmter Zylinder) sowie nach dem Aufbau ihrer Zellwand (grampositiv, gramnegativ) unterschieden werden. Feinere Unterteilungen tragen auch der Physiologie der Organismen Rechnung. So existieren aerobe, anaerobe sowie fakultativ anaerobe Bakterien. Manche Individuen sind in ihrer Eigenschaft als pathogene Keime von medizinischer Bedeutung, andere wiederum sind vollkommen harmlos.

Gegen Bakterien wirksame Substanzen sind seit geraumer Zeit bekannt. Der Begriff "Antibiotika" beispielsweise, der nicht auf alle antimikrobiell wirksamen Substanzen anwendbar ist, läßt sich auf das Jahr 1941 datieren, obwohl die ersten Erkenntnisse zum Penicillin bereits im Jahre 1929 gefunden wurden. Antibiotika im heutigen Sinne sind nicht für alle medizinischen, schon gar nicht kosmetische Anwendungen geeignet, da häufig auch der warmblütige Organismus, also etwa der erkrankte Patient, bei Anwendung auf irgendeine Weise in seinen Stoffwechselfunktionen beeinträchtigt wird.

Eine Aufgabe der vorliegenden Erfindung war also, den Stand der Technik in dieser Richtung zu bereichern, insbesondere also, Substanzen zur Verfügung zu stellen, welche gegen grampositive und/oder gramnegative Bakterien wirksam sind, ohne daß mit der Anwendung der Substanzen eine unvertretbare Beeinträchtigung der Gesundheit des Anwenders verbunden wäre.

Gramnegative Keime sind beispielsweise Escherichia coli, Pseudomonas-Arten sowie Enterobacteriaceen, wie etwa Citrobacter freundii.

Auch grampositive Keime spielen in Kosmetik und Dermatologie eine Rolle. Bei der unreinen Haut beispielsweise sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Eine besondere Aufgabe der vorliegenden Erfindung war es also, einen gegen unreine Haut bzw. Propionibacterium acnes wirksamen Stoff bzw. Stoffkombination zu finden.

Die vorliegende Erfindung betrifft in einer weiteren Ausführungsform kosmetische Desodorantien. Solche Formulierungen dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Mittel, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Eine weitere Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Pilze, auch Fungi [fungus = lat. Pilz], Mycota [µυκηζ = grch. Pilz] oder Mycobionten genannt, zählen im Gegensatze zu den Bakterien zu den Eucaryonten. Eucaryonten sind Lebewesen, deren Zellen (Eucyten) im Gegensatz zu denen der sogenannten Procaryonten (Procyten) über einen durch Kernhülle und Kernmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehltau (Phycomycetes) und die Ständerpilze (Basidiomycetes).

Pilze, auch nicht die Basidiomyceten, sind keine pflanzlichen Organismen, haben aber wie diese eine Zellwand, zellsaftgefüllte Vakuolen und eine mikroskopisch gut sichtbare Plasmaströmung. Sie enthalten keine photosynthetischen Pigmente und sind C-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophytien (z.B. Epidermophytie, Favus, Mikrosporie, Trichophytie), Hefemycosen (z.B. Pityriasis und andere Pityrosporum-bedingte Mycosen, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose und Skopulariopsidose), Systemmycosen (z.B. Chromomycose, Coccidiomycose, Histoplasmose).

Zu den pathogenen und fakultativ pathogenen Keimen gehören beispielsweise aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen. Eine Beteiligung von Pityrosporum ovale an der Entstehung von Psoriasis wird von der Fachwelt diskutiert.

Alle Bereiche der menschlichen Haut können von Dermatomycosen befallen werden. Dermatophytien befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche.

Ferner sind Superinfektionen der Haut durch Pilze und Bakterien nicht selten.

Bei bestehendem Primärinfekt, d.h., der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten -unschädliche, aber unter Umständen auch ausgesprochen pathogene Keime überwuchem auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nenneswerte Einbußen erleidet.

Im Gegensatze zu den prokaryotischen und eukaryotischen zellulären Organismen sind Viren [virus = lat. Gift] biologische Strukturen, welche zur Biosynthese eine Wirtszelle benötigen. Extrazelluläre Viren (auch "Virionen" genannt) bestehen aus einer ein- oder doppelsträngigen Nukleinsäuresequenz (DNS oder RNS) und einem Proteinmantel (dapsid genannt), gegebenenfalls einer zusätzlichen lipidhaltigen Hülle (Envelope) umgeben. Die Gesamtheit aus Nukleinsäure und Capsid wird auch Nucleocapsid genannt. Die Klassifikation der Viren erfolgte klassisch nach klinischen Kriterien, heutzutage allerdings zumeist nach ihrer Struktur, ihrer Morphologie, insbesondere aber nach der Nukleinsäuresequenz.

Medizinisch wichtige Virengattungen sind beispielsweise Influenzaviren (Familie der Orthomyxoviridae), Lyssaviren (z.B. Tollwut, Familie der Rhabdoviren) Enteroviren (z.B. Hepatitis-A, Familie der Picomaviridae), Hepadnaviren (z.B. Hepatitis-B, Familie der Hepadnaviridae).

Viruzide, also Viren abtötende Substanzen im eigentlichen Sinne gibt es nicht, da Viren nicht über eigenen Stoffwechsel verfügen. Es wurde aus diesem Grunde auch diskutiert, ob Viren als Lebewesen eingeordnet werden sollten. Pharmakologische Eingriffe ohne Schädigung der nicht befallenen Zellen ist jedenfalls schwierig. Mögliche Wirkmechanismen im Kampfe gegen die Viren sind in erster Linie die Störung deren Replikation, z.B. durch Blockieren der für die Replikation wichtigen Enzyme, die in der Wirtszelle vorliegen. Ferner kann das Freisetzen der viralen Nukleinsäuren in die Wirtszelle verhindert werden. Im Rahmen der hiermit vorgelegten Offenbarung wird unter Begriffen wie "antiviral" oder "gegen Viren wirksam", "viruzid" oder ähnlichen die Eigenschaft einer Substanz verstanden, einen ein- oder mehrzelligen Organismus vor schädlichen Folgen einer Virusinfektion, sei es prophylaktisch oder therapeutisch, zu schützen, ungeachtet dessen, was der tatsächliche Wirkmechanismus der Substanz im Einzelfalle sei.

Dem Stande der Technik mangelt es jedoch an gegen Viren wirksamen Substanzen, welche zudem den Wirtsorganismus nicht oder nicht in vertretbarem Maße schädigen.

Eine Aufgabe der vorliegenden Erfindung war also, diesem Übelstande abzuhelfen, also Substanzen zu finden, welche wirksam einen ein- oder mehrzelligen Organismus vor schädlichen Folgen einer Virusinfektion, sei es prophylaktisch oder therapeutisch, zu schützen.

Es wurde überraschend gefunden, und darin liegt die Lösung dieser Aufgabe, daß
(1) monomere oder homo- oder heterodimere oder homo- oder heterotrimere oder homo- oder heterotetramere Peptide, wobei diesen Peptiden als Homo- bzw. Heteromonomeres eine Struktur oder ein Strukturmotiv 
1                    10                    20                    30                    40
G I G D P V T X L K S G A I X H P V F X P R R Y K Q I G G X G L P X T K X X X X,
 zugrundeliegt, wobei X beliebig gewählt wird aus der Gruppe der essentiellen und nichtessentiellen Aminosäuren,
(2) oder wobei in den monomeren oder homo- oder heterodimeren oder homo-oder heterotrimeren oder homo- oder heterotetrameren Peptiden Strukturen wie vorgenannt vorliegen mit dem Unterschiede, daß bis zu fünf beliebige Aminosäuren fehlen, bis zu fünf beliebige Amonosäuren durch bis zu fünf beliebige andere Aminosäure ersetzt sind, oder an bis zu fünf beliebigen Stellen bis zu fünf weitere beliebige Aminosäuren vorgesehen sind,
(3) oder wobei in den monomeren oder homo- oder heterodimeren oder homo-oder heterotrimeren oder homo- oder heterotetrameren Peptiden Strukturen wie vorgenannt vorliegen mit dem Unterschiede, daß der C-Terminus und/oder der N-Terminus um bis zu 17 Aminosäuren deletiert und/oder ergänzt ist,
(4) oder wobei in monomeren oder homo-/hetero-di-, -tri-, oder tetrameren Peptiden oder Proteinen mit einem Molekulargewicht zwischen 10 und 100 kDalton unter den Punkten (1), (2) und (3) angegebenen Aminosäuresequenzen als einfach vorliegende oder sich mehrfach wiederholende Strukturmotive auftreten,
den Nachteilen des Standes der Technik abhelfen.

Als besondere Ausführungsformen der vorliegenden Erfindung werden kosmetische oder pharmazeutische Zubereitungen angesehen, welche sich durch einen wirksamen Gehalt an
(1) einem oder mehreren monomeren oder homo- oder heterodimeren oder homo-oder heterotrimeren oder homo- oder heterotetrameren Peptiden auszeichnen, wobei diesen Peptiden als Homo- bzw. Heteromonomeres eine Struktur 
1 10 20 30 40
G I G D P V T X L K S G A I X H P V F X P R R Y K Q I G G X G L P X T K X X X X,
 zugrundeliegt, wobei X beliebig gewählt wird aus der Gruppe der essentiellen und nichtessentiellen Aminosäuren,
(2) oder wobei in den monomeren oder homo- oder heterodimeren oder homo-oder heterotrimeren oder homo- oder heterotetrameren Peptiden Strukturen wie vorgenannt vorliegen mit dem Unterschiede, daß bis zu fünf beliebige Aminosäuren fehlen, bis zu fünf beliebige Amonosäuren durch bis zu fünf beliebige andere Aminosäure ersetzt sind, oder an bis zu fünf beliebigen Stellen bis zu fünf weitere beliebige Aminosäuren vorgesehen sind,
(3) oder wobei in den monomeren oder homo- oder heterodimeren oder homo-oder heterotrimeren oder homo- oder heterotetrameren Peptiden Strukturen wie vorgenannt vorliegen mit dem Unterschiede, daß der C-Terminus und/oder der N-Terminus um bis zu 17 Aminosäuren deletiert und/oder ergänzt ist
(4) oder wobei in monomeren oder homo-/hetero-di-, -tri-, oder tetrameren Peptiden oder Proteinen mit einem Molekulargewicht zwischen 10 und 100 kDalton unter den Punkten (1), (2) und (3) angegebenen Aminosäuresequenzen als einfach vorliegende oder sich mehrfach wiederholende Strukturmotive auftreten.

In der Tabelle 1 werden den essentiellen Aminosäuren ihre entsprechenden Drei- und Einbuchstabencodes gegenübergestellt. Der Einfachheit halber verwenden wir für die Charakterisierung der erfindungsgemäßen Strukturen die Einbuchstabencodes.

**Tabelle 1:**

| Drei- und Einbuchstabencodes für Aminosäuren | | |
|---|---|---|
| Aminosäure | | |
| L-Alanin | Ala | A |
| L-Valin | Val | V |
| L-Leucin | Leu | L |
| L-lsoleucin | Ile | I |
| L-Prolin | Pro | P |
| L-Tryptophan | Typ | W |
| L-Phenylalanin | Phe | F |
| L-Methionin | Met | M |
| Glycin | Gly | G |
| L-Serin | Ser | S |
| L-Tyrosin | Tyr | Y |
| L-Threonin | Thr | T |
| L-Cystein | Cys | C |
| L-Asparagin | Asn | N |
| L-Glutamin | Gln | Q |
| L-Asparaginsäure | Asp | D |
| L-Glutaminsäure | Glu | E |
| L-Lysin | Lys | K |
| L-Arginin | Arg | R |
| L-Histidin | His | H |

Dabei können das L-Selenocystein das L-Cystein und das L-Selenomethionin das L-Methionin ersetzen. Darüberhinaus können einzelne, mehrere oder auch alle Positionen auch durch die entsprechend D-konfigurierten Stereoisomere ersetzt werden.

Als ganz besonders bevorzugtes Peptid bzw. Peptide werden monomere oder homo- oder heterodimere oder homo- oder heterotrimere oder homo- oder heterotetramere Peptide, wobei diesen Peptiden als Homo- bzw. Heteromonomeres eine Struktur oder ein Strukturmotiv 
1 10 20 30 40
G I G D P V T C L K S G A I C H P V F C P R R Y K Q I G T C G L P G T T C C K K P
 zugrundeliegt, angesehen.

Als eine vorteilhafte Ausführungsform werden solche Peptide bzw. Zubereitungen, solche Peptide enthaltend angesehen, welche aus der Haut von Vertebraten, beispielsweise durch Extraktionsverfahren, erhältlich sind. Dabei ist dem Fachmanne klar, daß solche Peptide, auch auf anderem Wege synthetisch zugängig sind, beispielsweise durch chemische Synthese oder biotechnologische Verfahren.

Erfindungsgemäß ist auch die Verwendung solcher Wirkstoffe als antibakterielle, antimycotische oder antivirale Wirkstoffe. Ganz besonders vorteilhaft ist die Verwendung der erfindungsgemäßen Wirkstoffe als gegen grampositive Bakterien wirksame Wirkstoffe.

Es hat sich in erstaunlicher Weise herausgestellt, daß die erfindungsgemäß verwendeten Wirkstoffe das Wachstum von grampositiven und gramnegativen Bakterien, Mycobionten sowie Viren verhindern.

Insbesondere sind die erfindungsgemäß verwendeten Wirkstoffe befähigt, daß Wachstum von Hefen, insbesondere der Pityrosporum-Arten, namentlich Pityrosporum ovale, zu verhindern.

Es hat sich ferner herausgestellt, daß die erfindungsgemäß verwendeten Wirkstoffe die Bildung von seborrhoischen Erscheinungen, insbesondere Kopfschuppen, verhindern sowie bereits vorhandene seborrhoische Erscheinungen, insbesondere Kopfschuppen, zu beseitigen.

Die erfindungsgemäß verwendeten Wirkstoffe eignen sich darüberhinaus gut für die Verwendung als desodorierender Wirkstoff in kosmetischen Desodorantien sowie gegen unreine Haut, leichte Formen der Akne bzw. Propionibakterium acnes.

Die erfindungsgemäßen Wirkstoffe haben sich ebenfalls als besonders wirkungsvoll gegen Streptokokken erwiesen.

Schließlich hat sich herausgestellt, daß die erfindungsgemäß verwendeten Wirkstoffe den Verderb organischer Substanz, insbesondere kosmetischer und dermatologischer Zubereitungen, durch den Befall mit grampositiven und gramnegativen Bakterien, Mycobionten und Viren verhindern können, wenn sie diesen Zubereitungen zugesetzt werden.

Erfindungsgemäß sind somit auch ein Verfahren zur Bekämpfung von Mycobionten, dadurch gekennzeichnet, daß die erfindungsgemäß verwendeten Wirkstoffe gegebenenfalls in einem geeigneten kosmetischen oder dermatologischen Träger, mit dem durch Mycobionten kontaminierten Bereich in Kontakt gebracht werden, sowie ein Verfahren zum Schutze organischer Produkte vor dem Befall mit Mycobionten, dadurch gekennzeichnet, daß diesen organischen Produkten die erfindungsgemäß verwendeten Wirkstoffe in wirksamer Menge zugegeben werden.

Der Stand der Technik lieferte folglich nicht den geringsten Hinweis auf die erfindungsgemäße Verwendung als antimycotisches Wirkprinzip.

Ferner war erstaunlich, daß die die erfindungsgemäß verwendeten Wirkstoffe besonders gut wirksam sind gegen den für das Entstehen von Kopfschuppen verantwortlichen Keim Pityrosporum ovale und verwandte Keime. Eine bevorzugte Ausführungsform der vorliegenden Erfindung sind mithin gegen Kopfschuppen anzuwendende Formulierungen, beispielsweise Antischuppenshampoos.

Erfindungsgemäß werden die Wirkstoffe bevorzugt in kosmetischen oder dermatologischen Zusammensetzungen eingesetzt einem Gehalt von 0,0005 - 50,0 Gew.-%, insbesondere 0,01 - 20,0 Gew.-%, bezogen auf das Gesamtgewicht der Zusammensetzung bevorzugt sind. Vorteilhaft enthalten die Zusammensetzungen 0,02 - 10,0 Gew.-%, besonders bevorzugt 0,02 - 5,0 Gew.-% an den erfindungsgemäß verwendeten Wirkstoffen, ganz besonders vorteilhaft 0,5 - 3,0 Gew.-%, jeweils bezogen auf das Gesamtgewicht der Zusammensetzung.

Die erfindungsgemäß verwendeten Wirkstoffe lassen sich ohne Schwierigkeiten in gängige kosmetische oder dermatologische Formulierungen einarbeiten, vorteilhaft in Pumpsprays, Aerosolsprays, Cremes, Salben, Tinkturen, Lotionen, Nagelpflegeprodukte (z.B. Nagellacke, Nagellackentferner, Nagelbalsame) und dergleichen.

Es ist auch möglich und gegebenenfalls vorteilhaft, die erfindungsgemäß verwendeten Wirkstoffe mit anderen Wirkstoffen zu kombinieren, beispielsweise mit anderen antimikrobiell, antimycotisch bzw. antiviral wirksamen Stoffen.

Es ist vorteilhaft, die erfindungsgemäßen Zusammensetzungen abzupuffern. Vorteilhaft ist ein pH-Bereich von 3,5 - 7,5. Besonders günstig ist es, den pH-Wert in einem Bereich von 4,0 - 6,5 zu wählen.

Die erfindungsgemäßen kosmetischen und/oder dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung werden die erfindungsgemäßen kosmetischen und/oder dermatologischen Formulierungen in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdikkungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -mono-butylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl-und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe (FKW) und Fluorchlorkohlenwassertoffe (FCKW).

Bevorzugt können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure-(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bomylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Erfindungsgemäße kosmetische und/oder dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisierund Behandlungslotion, einen Haarlack oder um Dauerwellmittel.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel oder eine Wasch-, Duschoder Badezubereitung darstellen, enthalten vorzugsweise mindestens eine anionische, nicht-ionische oder amphotere oberflächenaktive Substanz oder Gemische daraus, erfindungsgemäße Wirkstoffe und Hilfsmittel, wie sie üblicherweise dafür verwendet werden.

Beispiele für oberflächen aktive Substanzen, die erfindungsgemäß vorteilhaft verwendet werden können, sind herkömmliche Seifen, z.B. Fettsäuresalze des Natriums, Alkylsulfate, Alkylethersulfate, Alkan- und Alkylbenzolsulfonate, Sulfoacetate , Sulfobetaine, Sarcosinate, Amidosulfobetaine, Sulfosuccinate, Sulfobernsteinsäurehalbester, Alkylethercarboxylate, Eiweiß-Fettsäure-Kondensate, Alkylbetaine und Amidobetaine, Fettsäurealkanolamide, Polyglycolether-Derivate.

Die oberflächenaktive Substanz kann in einer Konzentration zwischen 1 Gew.-% und 50 Gew.-% in dem Shampoonierungsmittel, bzw. der Wasch-, Dusch- oder Badezubereitung, vorliegen.

Liegt die kosmetische oder dermatologische Zubereitung in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetische oder dermatologische Zubereitung kann auch ein Aerosol mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische davon, sowie erfindungsgemäße Wirkstoffe. Die Menge der verwendeten erfindungsgemäßen Wirkstoffe liegt z.B. zwischen 0,1 und 10 Gew.-%, bevorzugt zwischen 0,1 und 3 Gew.-%.

Kosmetische und dermatologische Zubereitungen zur Behandlung und Pflege der Haare, die die erfindungsgemäß verwendeten Wirkstoffe enthalten, können als Emulsionen vorliegen, die vom nicht-ionischen oder anionischen Typ sind. Nicht-ionische Emulsionen enthalten neben Wasser Öle oder Fettalkohole, die beispielsweise polyethoxyliert oder polypropoxyliert sein können, oder auch Gemische aus den beiden organischen Komponenten. Diese Emulsionen enthalten gegebenenfalls kationische oberflächenaktive Substanzen.

Kosmetische und dermatologische Zubereitungen zur Behandlung und Pflege der Haare können als Gele vorliegen, die neben erfindungsgemäß verwendeten Wirkstoffen und dafür üblicherweise verwendeten Lösungsmitteln noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglykolstearat oder -distearat, enthalten. Das Verdickungsmittel ist im Gel z.B. in einer Menge zwischen 0,1 und 30 Gew.-%, bevorzugt zwischen 0,5 und 15 Gew.-%, enthalten.

Vorzugsweise beträgt die Menge der erfindungsgemäß verwendeten Wirkstoffe in einem für die Haare bestimmten Mittel 0,01 Gew.-% bis 10 Gew.%, insbesondere 0,5 Gew.-% bis 5 Gew.-%, bezogen auf das Gesamtgewicht der Zubereitungen.

Die folgenden Beispiele sollen die Verkörperungen der vorliegenden Erfindungen verdeutlichen. Die Angaben beziehen sich stets auf Gewichts-%, sofern nicht andere Angaben gemacht werden.

Da erfindungsgemäße Peptide aus der Haut von Vertebraten gewonnen werden können, unter anderem aus menschlicher Haut, soll hier und nachfolgend für solche Peptide das Kürzel "SAP" (= skin antimikrobial peptide) angewandt werden.

### Verfahren zur Isolierung von SAP aus menschlicher Haut

50 g Schuppen aus der Haut von Psoriatikern wurden mit Hilfe eines Dispergiergerätes in 0,1 mol/l Citrat-Puffer, pH 3,0, zerkleinert. Nach Zugabe von 30 Vol.% Ethanol wurde die Mischung zentrifugiert und der klare Überstand gegen phosphatgepufferte Kochsalzlösung (PBS), pH 7,4, über Amicon YM-3-Filter (cut off: 3kDa) diafiltriert. Nach Zentrifugieren wurde der klare Überstand auf eine mit Maus-Immunoglobulin kovalent beschichtete Affinitätssäule (HiTRAP, NHS-activated, 1ml, Pharmacia AB) gegeben und die Säule mit 2 ml PBS gewaschen.

Gebundene Proteine wurden zunächst mit 10 ml 2M Kochsalzlösung und anschließend mit 10 ml 0,2 M Glycin/HCl-Lösung (pH 2,0) eluiert.

Das NaCI-Eluat und das Glycin/HCI-Eluat wurden vereinigt und über einen Amicon YM-3-Filter gegen 0,1 % Trifluoressigsäure in Wasser zunächst diafiltriert und anschließend konzentriert. Danach wurde das Konzentrat auf eine präparative reversed-phase HPLC-Säule (RP-8, 250 mm x 12,5 mm, 300-7 C8, Macherey und Nagel) gegeben und mit einem linearen Gradienten ansteigender Konzentrationen an Acetonitril in 0,1 % Trifluoressigsäure/Wasser chromatographiert. Die Absorptione des Säulen-Eluates wurde bei 220 nm gemessen. Fraktionen, die mit 32-36 % Acetonitril eluiert wurden, wurden vereinigt und an einer Mikrobore-Mono S-HPLC-Säule (Smart-Mikro-HPLC-Apparat, Pharmacia AB), die vorher mit 50 mmol/l Ammoniumformiat äquilibiriert worden war, weiter aufgetrennt.

Proteine wurden mit Hilfe eines ansteigenden NaCl-Gradienten (Maximum: 1 Mol/l) im Äquilibrierungspuffer eluiert. Die im Bereich 0,48 Mol/i bis 0,55 Mol/l eluierbaren Proteine wurden gesammelt und abschließend einer Mikrobore-RP-18 HPLC-Trennung unterzogen.

Das bei 39 % Acetonitril eluierbare Protein wurde aufgefangen. Eine SDS-PAGE-Analyse mit Hilfe von High-Density-Gelen (Pharmacia AB) und dem Phast-Elektrophorese-System (Pharmacia AB) ergab nach Silberfärbung eine verbreiterte Bande entsprechend 15 kDa.

NH₂-terminale Squenzierungsversuche durch Edman-Abbau in einem Aminosäure-Analyser (Applied Biosystems) ergaben eine Primärstruktur

1 10 20 30 40
G I G D P V T C L K S G A I C H P V F C P R R Y K Q I G T C G L P G T T C C K K P

### Beispiel 1

| W/O-Lotion | |
|---|---|
| | I |
| Paraffinöl | 25,00 |
| Siliconöl | 2,00 |
| Ceresin | 1,50 |
| Wollwachsalkohol | 0,50 |
| Glucosesesquiisostearat | 2,50 |
| SAP | 50 mg/l |
| Parfum, Konservierungsstoffe | q.s. |
| Wasser, VES | ad 100,00 |
| pH: | ad 5,5 - 6,0 |

### Beispiel 2

| O/W-Crème | |
|---|---|
| | I |
| Pflanzliches Öl | 10,00 |
| Cetylalkohol | 2,00 |
| Glycerinmonostearat | 1,50 |
| PEG-30-Glycerylstearat | 2,00 |
| Glycerin | 3,00 |
| isopropylpalmitat | 5,00 |
| Carbopol 980 (neutralisiert) | 0,30 |
| SAP | 50 mg/l |
| Parfum, Konservierungsstoffe | q.s. |
| Wasser, VES | ad 100,00 |
| pH: | ad 5,5 - 6,0 |

### Beispiel 3

| Lippenstift | |
|---|---|
| | I |
| Ceresin | 8,00 |
| Bienenwachs | 4,00 |
| Carnaubawachs | 2,00 |
| Vaseline | 40,00 |
| Hydriertes Rizinusöl | 4,00 |
| Caprylic/Capric Triglyceride | 6,00 |
| SAP | 100 mg/l |
| Parfum, Konservierungsstoffe | q.s. |
| Wasser, VES | ad 100,00 |
| pH: | ad 5,5 - 6,0 |

### Beispiel 4

| Liposomenhaltiges Gesichtspflegegel | |
|---|---|
| | Gew.-% |
| Lecithin | 6,00 |
| Pflanzliches Öl | 12,50 |
| Hydrolysiertes Kollagen | 2,00 |
| Xanthan Gum | 1,40 |
| Butylenglycol | 3,00 |
| SAP | 50 mg/l |
| Parfum, Konservierungsstoffe | q.s. |
| Wasser, VES | ad 100,00 |
| pH: | ad 5,5 - 6,0 |

### Beispiel 5

| Duschpräparat mit Rückfetter | |
|---|---|
| | Gew.-% |
| Cocoamidodiacetat | 10,00 |
| Natriumlaurylsulfat | 25,00 |
| Kalium Cocyl Hydrolysiertes Kollagen | 5,00 |
| Macadamianußöl | 5,00 |
| Natriumchlorid | 0,60 |
| SAP | 150 mg/l |
| Parfum, Konservierungsstoffe | q.s. |
| Wasser, VES | ad 100,00 |
| pH: | ad 5,5 - 6,0 |
| pH: | ad 5,5 - 6,0 |

### Beispiel 6

| Syndetseife | |
|---|---|
| | Gew.-% |
| Natriumlaurylsulfat | 30,00 |
| Natriumsulfosuccinat | 10,00 |
| Kaliumcocoyl hydrolysiertes Kollagen | 2,00 |
| Dimethicon Copolyol | 2,00 |
| Paraffin | 2,00 |
| Maisstärke | 10,00 |
| Talcum | 10,00 |
| Glycerin | 3,00 |
| SAP | 75 mg/l |
| Parfum, Konservierungsstoffe | q.s. |
| Wasser, VES | ad 100,00 |
| pH: | ad 5,5 - 6,0 |
| pH: | ad 5,5 - 6,0 |

### Beispiel 7

| Pflegeshampoo | |
|---|---|
| | Gew.-% |
| Natriumlaurylsulfat | 34,00 |
| Dinatriumlaurylsulfosuccinat | 6,00 |
| Cocoamidopropylbetain | 10,00 |
| Glycoldistearat | 5,00 |
| SAP | 50 mg/l |
| Parfum, Konservierungsstoffe | q.s. |
| Wasser, VES | ad 100,00 |
| pH: | ad 5,5 - 6,0 |
| pH: | ad 5,5 - 6,0 |

### Beispiel 8

| Haarspülung | |
|---|---|
| | Gew.-% |
| Cocoamidopropylbetain | 5,00 |
| Cetylalkohol | 2,00 |
| Propylenglycol | 2,00 |
| SAP | 100 mg/l |
| Parfum, Konservierungsstoffe | q.s. |
| Wasser, VES | ad 100,00 |
| pH: | ad 5,5 - 6,0 |
| pH: | ad 5,5 - 6,0 |
| pH: | ad 5,5 - 6,0 |

### Beispiel 9

| Fußcrème | |
|---|---|
| | Gew.-% |
| Soluan 5 | 2,00 |
| Methylsalicylat | 5,00 |
| Caprylic/Capric Triglyceride | 10,00 |
| Stearinsäure | 5,00 |
| Cetylalkohol | 1,00 |
| Glycerin | 2,00 |
| Dimethicon | 1,00 |
| Carbopol 984 | 0,50 |
| Triethanolamin | 1,50 |
| SAP | 100 mg/l |
| Parfum, Konservierungsstoffe | q.s. |
| Wasser, VES | ad 100,00 |
| pH: | ad 5,5 - 6,0 |
| pH: | ad 5,5 - 6,0 |
| pH: | ad 5,5 - 6,0 |

### Beispiel 10

| Deo-Pumpspray | |
|---|---|
| | Gew.-% |
| PEG-40-Hydriertes Rizinusöl | 2,00 |
| Glycerin | 1,00 |
| SAP | 50 mg/l |
| Parfum, Konservierungsstoffe | q.s. |
| Wasser, VES | ad 100,00 |
| pH: | ad 5,5 - 6,0 |
| pH: | ad 5,5 - 6,0 |
| pH: | ad 5,5 - 6,0 |

## Patentansprüche

1. Monomere oder homo- oder heterodimere oder homo- oder heterotrimere oder homo- oder heterotetramere Peptide,
(1) wobei diesen Peptiden als Homo- bzw. Heteromonomeres eine Struktur oder ein Strukturmotiv 
1 10 20 30 40
G I G D P V T X L K S G A I X H P V F X P R R Y K Q I G G X G L P X T K X X X X,
 zugrundeliegt, wobei X beliebig gewählt wird aus der Gruppe der essentielten und nichtessentiellen Aminosäuren,
(2) oder wobei in den monomeren oder homo- oder heterodimeren oder homo-oder heterotrimeren oder homo- oder heterotetrameren Peptiden Strukturen wie vorgenannt vorliegen mit dem Unterschiede, daß bis zu fünf beliebige Aminosäuren fehlen, bis zu fünf beliebige Amonosäuren durch bis zu fünf beliebige andere Aminosäure ersetzt sind, oder an bis zu fünf beliebigen Stellen bis zu fünf weitere beliebige Aminosäuren vorgesehen sind,
(3) oder wobei in den monomeren oder homo- oder heterodimeren oder homo-oder heterotrimeren oder homo- oder heterotetrameren Peptiden Strukturen wie vorgenannt vorliegen mit dem Unterschiede, daß der C-Terminus und/oder der N-Tenninus um bis zu 17 Aminosäuren deletiert und/oder ergänzt ist,
(4) oder wobei in monomeren oder homo-/hetero-di-, -tri-, oder tetrameren Peptiden oder Proteinen mit einem Molekulargewicht zwischen 10 und 100 kDalton unter den Punkten (1), (2) und (3) angegebenen Aminosäuresequenzen als einfach vorliegende oder sich mehrfach wiederholende Strukturmotive auftreten.

2. Monomere oder homo- oder heterodimere oder homo- oder heterotrimere oder homo- oder heterotetramere Peptide nach Anspruch 1,
(1) wobei diesen Peptiden als Homo- bzw. Heteromonomeres eine Struktur oder ein Strukturmotiv 
1 10 20 30 40
G I G D P V T C L K S G A I C H P V F C P R R Y K Q I G T C G L P G T T C C K K P
(2) oder wobei in den monomeren oder homo- oder heterodimeren oder homo-oder heterotrimeren oder homo- oder heterotetrameren Peptiden Strukturen wie vorgenannt vorliegen mit dem Unterschiede, daß bis zu fünf beliebige Aminosäuren fehlen, bis zu fünf beliebige Amonosäuren durch bis zu fünf beliebige andere Aminosäure ersetzt sind, oder an bis zu fünf beliebigen Stellen bis zu fünf weitere beliebige Aminosäuren vorgesehen sind,
(3) oder wobei in den monomeren oder homo- oder heterodimeren oder homo-oder heterotrimeren oder homo- oder heterotetrameren Peptiden Strukturen wie vorgenannt vorliegen mit dem Unterschiede, daß der C-Terminus und/oder der N-Terminus um bis zu 17 Aminosäuren deletiert und/oder ergänzt ist,
(4) oder wobei in monomeren oder homo-/hetero-di-, -tri-, oder tetrameren Peptiden oder Proteinen mit einem Molekulargewicht zwischen 10 und 100 kDalton unter den Punkten (1), (2) und (3) angegebenen Aminosäuresequenzen als einfach vorliegende oder sich mehrfach wiederholende Strukturmotive auftreten,
zugrundeliegt.

3. Kosmetische oder pharmazeutische Zubereitungen, welche sich durch einen wirksamen Gehalt an einem oder mehreren monomeren oder homo- oder heterodimeren oder homo- oder heterotrimeren oder homo- oder heterotetrameren Peptiden nach Anspruch 1 oder 2 als Wirkstoff oder Wirkstoffe, auszeichnen.

4. Wirkstoffe gemäß Anspruch 1 oder 2 zur Verwendung als antimikrobielle, antimycotische oder antivirale Wirkstoffe.

5. Zubereitungen nach Anspruch 3, **dadurch gekennzeichnet, daß** der oder die Wirkstoffe eingesetzt werden in Gehalten von 0,0005 - 50,0 Gew.-%, insbesondere 0,01 - 20,0 Gew.-%, bevorzugt 0,02 - 5,0 Gew.-%, ganz besonders vorteilhaft 0,5 - 3,0 Gew.-%, jeweils bezogen auf das Gesamtgewicht der Zusammensetzung.

## Claims

1. Monomeric or homo- or heterodimeric or homo- or heterotrimeric or homo- or heterotetrameric peptides,
(1) these peptides being based as homo- or heteromonomer on a structure or a structural motif 
1          10          20          30          40
GIGDPVTXLKSGAIXHPVFXPRRYKQIGGXGLPXTKXXXX,
 X being selected at will from the group consisting of the essential and non-essential amino acids,
(2) or, in the monomeric or homo- or heterodimeric or homo- or heterotrimeric or homo- or heterotetrameric peptides, structures such as the abovementioned being present with the difference that up to any five desired amino acids are absent, up to any five desired amino acids are replaced by up to any five other desired amino acids, or in up to any five desired sites up to any five further desired amino acids are present,
(3) or, in the monomeric or homo- or heterodimeric or homo- or heterotrimeric or homo- or heterotetrameric peptides, structures such as the abovementioned being present with the difference that up to 17 amino acids are deleted from and/or added to the C terminus and/or the N terminus,
(4) or, in monomeric or homo-/hetero-di-, -tri- or-tetrameric peptides or proteins having a molecular weight between 10 and 100 kDaltons, amino acid sequences indicated under items (1), (2) and (3) occurring as structural motifs present singly or recurring several times.

2. Monomeric or homo- or heterodimeric or homo- or heterotrimeric or homo- or heterotetrameric peptides according to Claim 1,
(1) these peptides being based as homo- or heteromonomer on a structure or a structural motif 
1          10         20         30         40
GIGDPVTCLKSGAICHPVFCPRRYKQIGTCGLPGTTCCKKP
 X being selected at will from the group consisting of the essential and non-essential amino acids,
(2) or, in the monomeric or homo- or heterodimeric or homo- or heterotrimeric or homo- or heterotetrameric peptides, structures such as the abovementioned being present with the difference that up to any five desired amino acids are absent, up to any five desired amino acids are replaced by up to any five other desired amino acids, or in up to any five desired sites up to any five further desired amino acids are present,
(3) or, in the monomeric or homo- or heterodimeric or homo- or heterotrimeric or homo- or heterotetrameric peptides, structures such as the abovementioned being present with the difference that up to 17 amino acids are deleted from and/or added to the C terminus and/or the N terminus,
(4) or, in monomeric or homo-/hetero-di-, -tri- or-tetrameric peptides or proteins having a molecular weight between 10 and 100 kDaltons, amino acid sequences indicated under items (1), (2) and (3) occurring as structural motifs present singly or recurring several times.

3. Cosmetic or pharmaceutical preparations which are distinguished by an efficacious content of one or more monomeric or homo- or heterodimetic or homo- or heterotrimeric or homo- or heterotetrameric peptides according to Claim 1 or Claim 2 as active ingredient or active ingredients.

4. Active ingredients according to Claim 1 or 2 for use as antimicrobial, antimycotic or antiviral active ingredients.

5. Preparations according to Claim 3, **characterized in that** the active ingredient(s) is/are employed in contents of 0.0005 - 50.0% by weight, in particular 0.01 - 20.0% by weight, preferably 0.02 - 5.0% by weight, very particularly advantageously 0.5 - 3.0% by weight, in each case based on the total weight of the composition.

## Revendications

1. Peptides monomères ou homo- ou hétérodimères ou homo- ou hétérotrimères ou homo- ou hétérotétramères
(1) une structure ou un motif structural 
1                10                20                30                40
GIGDPVTXLKSGAIXHPVFXPRRYKQIGGXGLPXTKXXXX,
 en tant qu'homo- ou hétéromonomère, étant à la base de ces peptides, X étant choisi de façon quelconque dans le groupe des aminoacides essentiels ou non essentiels,
(2) ou des structures comme indiqué précédemment étant présentes dans les peptides monomères ou homo- ou hétérodimères ou homo- ou hétérotrimères ou homo- ou hétérotétramères, avec la différence que jusqu'à cinq aminoacides quelconques sont absents, jusqu'à cinq aminoacides quelconques sont remplacés par jusqu'à cinq autres aminoacides quelconques, ou jusqu'à cinq aminoacides quelconques supplémentaires sont prévus en jusqu'à cinq positions quelconques,
(3) ou des structures comme indiqué précédemment étant présentes dans les peptides monomères ou homo- ou hétérodimères ou homo- ou hétérotrimères ou homo- ou hétérotétramères, avec la différence que l'extrémité C-terminale et/ou l'extrémité N-terminale est privée de et/ou complétée avec jusqu'à 17 aminoacides,
(4) ou, dans des protéines ou peptides monomères ou homo-/hétérodimères, -trimères ou -tétramères ayant une masse moléculaire comprise entre 10 et 100 kilodaltons, des séquences d'aminoacides indiquées aux points (1), (2) et (3) apparaissant en tant que motifs structuraux présents une fois ou se répétant plusieurs fois.

2. Peptides monomères ou homo- ou hétérodimères ou homo- ou hétérotrimères ou homo- ou hétérotétramères selon la revendication 1,
(1) une structure ou un motif structural 
1                    10                    20                    30                    40
GIGDPVTCLKSGAICHPVFCPRRYKQIGTCGLPGTTCCKKP
 en tant qu'homo- ou hétéromonomère, étant à la base de ces peptides,
(2) ou des structures comme indiqué précédemment étant présentes dans les peptides monomères ou homo- ou hétérodimères ou homo- ou hétérotrimères ou homo- ou hétérotétramères, avec la différence que jusqu'à cinq aminoacides quelconques sont absents, jusqu'à cinq aminoacides quelconques sont remplacés par jusqu'à cinq autres aminoacides quelconques, ou jusqu'à cinq aminoacides quelconques supplémentaires sont prévus en jusqu'à cinq positions quelconques,
(3) ou des structures comme indiqué précédemment étant présentes dans les peptides monomères ou homo- ou hétérodimères ou homo- ou hétérotrimères ou homo- ou hétérotétramères, avec la différence que l'extrémité C-terminale et/ou l'extrémité N-terminale est privée de et/ou complétée avec jusqu'à 17 aminoacides,
(4) ou, dans des protéines ou peptides monomères ou homo-/hétérodimères, -trimères ou -tétramères ayant une masse moléculaire comprise entre 10 et 100 kilodaltons, des séquences d'aminoacides indiquées aux points (1), (2) et (3) apparaissant en tant que des motifs structuraux présents une fois ou se répétant plusieurs fois.

3. Préparations cosmétiques ou pharmaceutiques, qui se distinguent par une teneur efficace en un ou plusieurs peptides monomères ou homo- ou hétérodimères ou homo- ou hétérotrimères ou homo- ou hétérotétramères selon la revendication 1 ou 2, en tant que substance active ou substances actives.

4. Substances actives selon la revendication 1 ou 2, en tant que substances actives antimicrobiennes, antimycosiques ou antivirales.

5. Préparations selon la revendication 3, **caractérisée en ce que** les substances actives sont utilisées à des concentrations de 0,0005 à 50,0% en poids, en particulier de 0,01 à 20,0% en poids, de préférence de 0,02 à 5,0% en poids, de façon tout particulièrement avantageuse, de 0,5 à 3,0% en poids, dans chaque cas par rapport au poids total de la composition.
